# EUROPEAN PATENT APPLICATION

(11) **EP 3 730 128 A1**
(43) Date of publication of application: **28.10.2020**
(21) Application number: 17935307.3
(22) Date of filing: 20.12.2017
(51) Int. Cl.: A61K 9/70

(54) **PASTING STRUCTURE**

(71) Applicant: Easting Biotech Company Limited, New Taipei City 241 (TW)
(72) Inventor: CHEN, Shih-wei, New Taipei City, Taiwan 221 (TW); KU, Chen-an, New Taipei City, Taiwan 221 (TW)
(74) Representative: Ter Meer Steinmeister & Partner
(86) International application number: PCT/CN2017/117429
(87) International publication number: WO 2019/119292

(57) **Abstract**

The present invention is related to an pasting structure that comprises a back-material layer which is formed by attaching the film layer and the porous supporting layer to each other; and a gel layer, one side of the gel layer is bonded to the porous supporting layer and part of the gel layer penetrate the pore; wherein the back-material layer and the gel layer closely adhered to each other to form a transparent pasting structure. The pasting structure of the present invention is used as wound dressing which easily observes the wound change and healing status at any time. In the meantime, that provides higher moisture permeability and comfort.

## Description

### Field of the Invention

The present invention is related to a pasting structure, in particular related to be transparent, which easily observes the wound change and healing status. In the meantime, the pasting structure provides higher moisture permeability and comfort.

### Background of the Invention

According to the science and statistic report in recent year, the demand of market of global wound is getting more and more. In particular, surgical trauma, the total number has been over 10 million person-times per year and it is increasing every year. Because of trauma and laceration by accidences, about 20 million person-times per year. The number is 10 million person times by burns per year. Ulcerative wounds caused by chronic diseases, diabetes, and aging population have exceeded 30 million person-times per year.

Because the need of the treatment and popularity of endoscopic surgery, the risk has been greatly reduced which caused by surgery. But subsequently the demand of better wound care and scar prevention after surgery. There are methods of wound care with various advanced pasting structure at the present and then which have been used to shorten the healing time and eliminate scars.

The traditional pasting structure comprises natural plant fibre or animal fur material, such as gauze, cotton pads, wool, various oil gauze, etc. This type of attachment is only temporary covering material and that often need to be alternated within a certain period of time. However, these wound pasting structures often are prone to stick with wound during the period time of replacement. Therefore, it is possible to tear neonatal epithelial cells or wounds that have been healed together while tearing pasting structure, and that is not good to the natural healing of the wound.

The research on the existing of pasting structure make people have understanding of attachment with science. That shows the better pasting structure can keep the favourable and healing environment for cell growth in the wound, controlling and absorbing exudate, breathable, moisture, preventing bacteria invasion, being closely attach individual, loading and released drug. On the other hand, it should have good tissue and blood compatibility, and adhesion and scaling should not occur when it is removed from the individual. It should also have better mechanical properties and tensile strength and be easy to use it.

Comparing with traditional pasting structure, hydrogel pasting structure can provide better breathability, moisture, block bacteria invasion and control and absorb exudate, etc., so that the wound is in the good cell growth healing environment. In addition, hydrogel pasting structure can attach the wound closely and that will not stick on the wound and cause second injury when it is alternated. Therefore, hydrogel pasting structure is widely used in the field of biomedicine.

Generally speaking, the supporting layer of the traditional pasting structure is composed of polyethylene (PE), polypropylene (PP) or nylon (Nylon). When it combined with hydrogel to form a pasting structure. Because the supporting layer cannot provide enough tensile strength and that cause the structure to break and make the structure or the product unable to fit user's body. As a result, the lack of compliance with user's body generally cause result in hydrogel pasting structure is not able to provide comfort and suitability required by users. In addition, the lack of compliance with the user's body generally results in the hydrogel pasting structure products not being able to effectively absorb, distribute and maintain the liquid. Another problem caused by the lack of easy stretching of the hydrogel pasting structure is that the structure may disintegrate into materials in isolated areas or groups, causing discomfort to the user and reducing the efficiency of the structure.

The research people in German recently invented a bandage that its color will turn purple in the early stage of wound infection. The new dressings used in this bandage developed by the German Munich Modular Solid-State Research Institute contain a special dye that reacts to different pH levels. This new external bandage can be used to isolate wound like others, but the new product provides the special method of observing the situation of wound healing. In general, healthy skin and wound healing are weak alkaline with pH around 5-6. pH rising, it tends to be alkaline, it indicates that the infection is occurring. If pH is between 6.5 and 8.5, the new bandage will turn purple. The color-changing is useful for patients and doctors to monitor the infection without alternating dressings. This is an advantage. Because removing the bandage from wound, bacteria will swoop in.

At present, the scientific community and dermatologists know that the optimal pH value in the process of wound healing should be weakly acidic, which can inhibit the growth of bacteria, shorten the inflammation period of the wound, and promote the regeneration of blood vessels and epithelial tissues, shorten the wound healing time. The Joanneum Institute of Austrian State Steinbahn announced that it has successfully developed a new generation of medical supplies, the "cotton swab" (Wattestäbchen), which can detect the pH value of the skin surface while cleaning the wound, which helps medical personnel to master healing and recovery progress and improve medical effects. The cotton swab developed by Joanneum is similar to a "traffic signal light". Due to the special stain, when the wound is treated with the cotton swab or cotton swab, if the skin is weakly acidic, it will be displayed in light green (green light) And when the wound skin is alkaline, a red "warning sign" will appear to remind the wounded or nursing staff to clean and change the dressing in time, which can save the time that needs additional sampling and testing in the past, and effectively improve the medical effect.

The long-term requirement in this field is to produce a hydrogel pasting structure that exceeds performance characteristics of well-known hydrogel structure. More specially, it also hopes that hydrogel pasting structure is able to tensile and be compliance with user's body. The hydrogel pasting structure can easily observe the wound change and healing status at any time under normal usage. In the meantime, that provides higher moisture permeability and comfort to wounds

### Detailed Description of the Invention

Therefore, inventors found hydrogel pasting structure can solve the question above mentioned through multi-time testing. The invention provides a pasting structure which easily observes the wound change and healing status at any time under normal usage. In the meantime, that provides higher moisture permeability and comfort. Including a back-material layer, which is formed by attaching a film layer and a porous supporting layer to each other; and a gel layer, which is bonded to the porous supporting layer, and part of the gel layer penetrates and fills the pores like the fusion of cement and reinforcing steel, wherein the back-material layer and the gel layer closely adhered to each other to form a transparent pasting structure.

The pasting structure of the present invention, a gel of the gel layer not limit to hydrogel or Acrylic Hydrogel.

The pasting structure of the present invention, a film layer comprises but not limited to Polyurethane (PU) layer, Polypropylene (PP) layer, Polyethylene (PE) layer, Polyethylene terephthalate (PET) layer or polymer material to form the film.

The pasting structure of the present invention, the porous supporting layer comprises but not limited to polypropylene, polyurethane, polyethylene, polyethylene terephthalate, Nylon, cotton wool or Rayon non-woven layer.

In one embodiment, the gel layer in the pasting structure of the present invention is hydrogel which coated or connected to the supporting layer after UV irradiation or temperature change and solidified to form the gel layer.

Transparency in the present invention refers to the property that allows light to pass through, which can range from a little opaque to fully transparent, and transparent materials are perspective; that mean they allow clear images to pass through. In a preferred embodiment, compared to 100% total light transmission, the term "transparent" refers to a light transmittance greater than 30%; in a preferred embodiment, the term "transparent" refers to a light transmittance greater than 50%. Translucent materials only allow light to pass through. Translucent materials only allow light to pass through. In a preferred embodiment, the backing layer is transparent or matte.

There is imprinting on the supporting layer in the pasting structure of the present invention, which embosses a specific type or symbol for identifying the source of the product.

The supported structure of the present invention, in another preferred embodiment, further sprayed porous materials on the film layer to design the patterns or texts and stick on the film layer for identifying the source of product or design patterns to enhance user's willing.

The present invention of pasting structure further comprises a release layer which attached to another side of the gel layer. In one embodiment, the release layer comprises but not limited to paper or film layer, in one preferred embodiment, the release layer is a polyurethane layer.

The gel layer in the present invention, in a preferred embodiment, as a contact surface for the wound, the gel layer can put the wound in the appropriated humidity state for accelerating healing. The wet healing environment is helpful to wound healing. If the wound is dehydrated, the cells cannot survive. Although various wet gauze or bondages also provide humidity healing environment, the gauze or bondage-like pasting structure need to be replaced frequently and result in being susceptible to damage for the new cells, causing the secondary injury to the wound and the risk of dehydration. The gel layer in the present invention has the characteristics of absorbing and debonding. when replacing the dressing, there is less damage to the wound than the general gauzes or bondages such as Vaseline gauze, and its absorbing rate is more suitable for the best moist state in wound healing need, absorbing excess water, keeping proper humidity of the skin and having more drug carrying.

∘ The gel layer in the present invention may contain an active ingredient, so the present invention can apply to various wound pasting structures, post-cutting pasting structure, electronic pasting structures, medicine containing adhesive tapes, facial patch or beauty patch, other covering daily necessities such as anti-mosquito stickers, heel stickers, etc. The beneficial effect of the present invention is that the pasting structure can have multidirectional elasticity for a variety of wounds, providing the better wound healing environment, reducing healing time, antibacterial and reducing the chance of infection.

The gel layer in the present invention may contains special dyes which reacts to different pH level, so the present invention can apply to various new utility wound dressings. When the pasting structures are used to treat wounds, and it will display in light green (green light) if the skin is weak acidic. When the wound skin is alkaline, the red 'warning' will appear and remind the wounded and nursing people can clean and replace the wound dressings in time, and that saves previously need for additional time of getting and testing samples, it can improve the medical effect.

The porous supporting layer in the present invention may coated with special dyes which reacts to different pH level and designed texts or patterns on it. Not only improving the medical effect above-mentioned but also further making texts or patterns with color-changing to identify the resource of the product or enhance user's wiliness by designing patterns.

### Brief Description of the Drawings

FIG.1 is schematic diagram of the pasting structure of the present invention.
FIG.2 is schematic diagram of the porous material sprayed on the film layer to produce words of 'EB'.

### Description of Embodiments

The preferred embodiment of the present invention will be described below for details, wherein the drawing numbers of the listed embodiments are same as the numbers shown in the drawings, please refer to the drawings and detailed description at the same time. The examples below are not limiting and merely are representative of some aspects and features of the present invention.

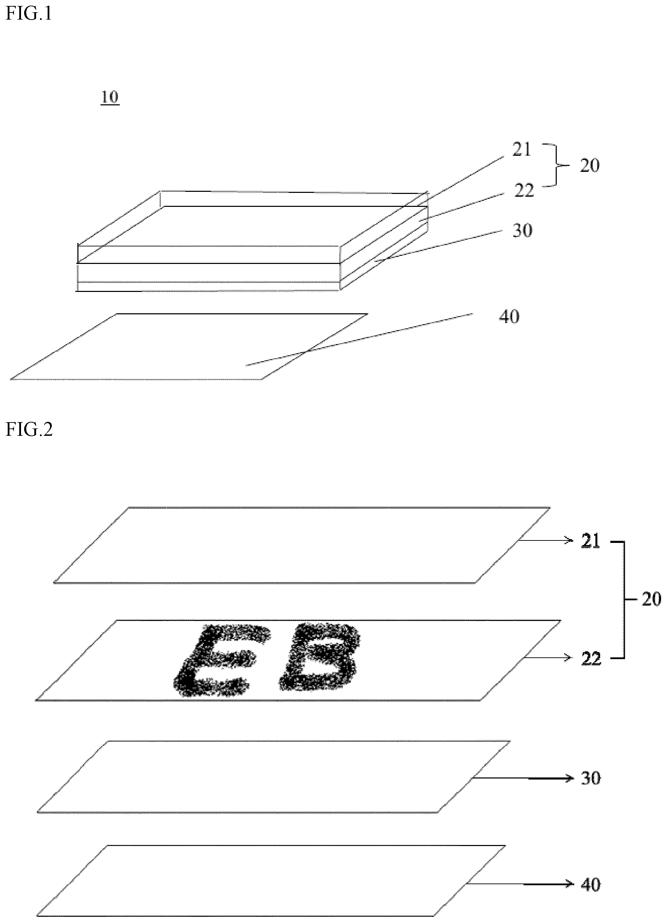

FIG.1 is schematic diagram of pasting structure of present invention, the pasting structure 10 of the present invention comprises: a back-material layer 20, which is formed by attaching the film layer 21 and the porous supporting layer 22 to each other; and a gel layer 30, one side of that combined with the porous supporting layer 22 and the part of the gel layer 30 penetrates the pores, wherein the back-material layer 20 and the gel layer 30 are closely adhere together to form a transparent pasting structure.

In a preferred embodiment, the porous supporting layer 22 , which is polypropylene non-woven fabric, that is attached the film layer 21 composed of polyurethane film with pressure sensitive adhesives become the back-material layer 20. The thickness of the polyurethane film is 0.01∼0.2 mm. The hydrogel is coated or soaked in the polypropylene non-woven fabric part of the back-material, the hydrogel fully penetrates into the polypropylene non-woven fabric and extend to a thickness and the thickness greater than non-woven fabric layer. The hydrogel solidified to form the gel layer 30 after UV irradiation or temperature change. The solidified gel penetrates into the pore structure of the non-woven fabric, both of them combined together. The gel layer 30 and the back-material layer 20 adhered closely as an adhesive layer and appear transparent. When it used as wound dressings, it will be easily observed the change and healing status of the wound. In addition, the polyurethane film is a kind of moisture permeable film, which can be applied as wound dressings on the wound and provide higher moisture permeability and comfort.

In a preferred embodiment, according to the porous supporting layer 22 of the pasting structure 10 of the present invention, there is imprinting on it which embossed a specific type or symbol for identifying the source of the product., or sprayed porous materials on the film layer to design patterns or texts and stick on the film layer to identify the source of product or o enhance user's willing by the texts and patterns design.

In a preferred embodiment, according to the pasting structure 10 of the present invention, the gel layer 30 may comprise a special dye which reacts to different pH level. When the pasting structures are used to treat wounds, and it will display in light green (green light) if the skin is weak acidic. When the wound skin is alkaline, the red 'warning' will appear and remind the wounded and nursing people to clean and replace the wound dressings in time, and that saves previously need for additional time of getting and testing samples, it can improve the medical effect.

In a more preferred embodiment, according to the pasting structure 10 of the present invention, the porous supporting layer 22 may coated with special dyes which react to different pH level and designed texts or patterns. That generates the source of the product to identify or enhances user's wiliness and remind the wounded and nursing people to clean and replace the wound dressings in time, and that saves previously need for additional time of getting and testing samples, it improves the medical effect.

According to the pasting structure 10 of present invention, it further comprises a release layer 40, which attached to the other side of the gel layer. In the present embodiment, the release layer 40 is composed of polyurethane (PU) and the gel layer is made of hydrogel.

Although the present invention is implemented as described above, it not limited to present invention. The person in the art can make changes and modifications within the spirit and the range in the present invention. Therefore, the protection scope of the present invention takes claims of the present specification as the reference.

## Claims

1. A pasting structure comprising:
a back-material layer which is formed by attaching the film layer and the porous supporting layer to each other; and
a gel layer, which is boned to the porous supporting layer and part of the gel layer penetrates the pore,
Wherein the back-material layer and the gel layer closely adhered to each other to form a pasting structure with a transmittance greater than 30%.

2. The pasting structure of claim 1, wherein the back-material layer and the gel layer are closely adhered to each other to form the pasting structure with a transmittance greater than 50%.

3. The pasting structure of claim 1, wherein the gel layer is formed by the hydrogel solidified.

4. The pasting structure of claim 1, wherein the gel layer comprises active ingredient.

5. The pasting structure of claim 1, wherein the gel layer comprises the special dyes which react to different pH level.

6. The pasting structure of claim 1, wherein the thickness of the film layer is 0.01∼0.2 mm.

7. The pasting structure of claim 1, wherein the supporting layer has imprinting.

8. The pasting structure of claim 1, further comprising a release layer which attached to another side of the gel layer.

9. The pasting structure of claim 8, wherein the release layer is paper or the film layer.

10. The pasting structure of claim 1, wherein the porous supporting layer sprayed on the film layer to design the patterns or texts and stick on the film layer
